(19) Europäisches Patentamt
European Patent Office
Office européen des brevets

(11) Publication number: **0 104 647**
**B1**

(12) # EUROPEAN PATENT SPECIFICATION

(45) Date of publication of patent specification: **30.03.88**

(21) Application number: **83109607.8**

(22) Date of filing: **27.09.83**

(51) Int. Cl.$^4$: **C 07 D 471/04** // A61K31/505, C07D211/34, C07D213/56, C07D213/57 ,(C07D471/04, 239:00, 221:00)

(54) **Pyridopyrimidinones.**

(30) Priority: **27.09.82 US 424936**
**14.09.83 US 532120**

(43) Date of publication of application:
**04.04.84 Bulletin 84/14**

(45) Publication of the grant of the patent:
**30.03.88 Bulletin 88/13**

(84) Designated Contracting States:
**BE CH DE FR GB IT LI NL SE**

(56) References cited:
**GB-A-1 114 397**

**Journal Med. Chem, vol. 28, 1985, pp.1285-1291**
**JOURNAL OF THE CHEMICAL SOCIETY PERKIN II, London, 1972, p. 1920-1925, T.A.CRABB et al. "Proton magnetic resonance studies of compounds with bridgehead nitrogen atoms. Part XXII. The stereochemistry and 1 H nuclear magnetic resonance spectra of some perhydro-2-methyl-imidazo (1,5-a)-pyridin-1-ones and perhydro-2-methylpyrido(1,2-c)pyrimidin-3-ones"**

(73) Proprietor: **G.D. Searle & Co.**
**P.O. Box 1045**
**Skokie Illinois 60076 (US)**

(72) Inventor: **Adelstein, Gilbert William**
**27588 Prairie Avenue**
**Evanston, IL 60201 (US)**
Inventor: **Chorvat, Robert John**
**815 East Waverly Drive**
**Arlington Heights, IL 60004 (US)**

(74) Representative: **Wolff, Hans Joachim, Dr.jur. Dipl.-Chem. et al**
**Beil, Wolff & Beil Rechtsanwälte**
**Postfach 80 01 40 Adelonstrasse 58**
**D-6230 Frankfurt am Main 80 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 104 647 B1

Courier Press, Leamington Spa, England.

**Description**

BACKGROUND OF THE INVENTION

(a) Field of the invention

This invention relates to novel pyridopyrimidinones useful as antiarrhythmic agents. In particular, the invention relates to novel pyridopyrimidinones of the Formula IV which are useful in the treatment of arrhythmic disorders.

Arrhythmias are disorders relating to impulse generation in the heart. The disorders include premature contractions (extrasystoles) originating in abnormal or ectopic foci in atria or ventricles, paroxysmal supraventricular tachycardia, atrial flutter, atrial fibrillation, and venticular tachycardia and fibrillation. For a discussion on these disorders see for example the Pharmacological Basis of Therapeutics, Goodman and Gilman, the MacMillan Co., pgs. 709—711 (1970).

A number of compounds have been developed to alter cardiovascular function related to heart rate and rhythm. The cardiac glycosides, including digitalis, have as their main pharmacodynamic property the ability to increase the force of myocardial contraction. The salutary effects of the drug in congestive heart failure — increased cardiac output, decreased heart size, venous pressure and blood volume; diuresis and relief of edema — are all explained on the basis of increased contractile force, a positive inotropic action. Quinidine is useful in the therapy of atrial fibrillation but exhibits several toxic reactions, such as cinchonism. The cardiac actions of procainamide are essentially identical to those for quinidine, and like quinidine, procainamide exhibits toxic side effects. Lidocaine, a widely used local anesthetic administered intravenously may be used in the treatment of ectopic ventricular rhythms, but is not recommended for the treatment of supraventricular arrhythmias. Propranolol has found use in the treatment of supraventricular tachycardias and ventricular arrhythmias but because propranolol antagonizes adrenergic effects on contractility and because serious arrthymias are commonly encountered in the presence of overt or incipient heart failure, the use of the drug for its anriarrhythmic actions demands great care. Disopyramide is similar to procainamide and quinidine (referred to as a Type 1 antiarrhythmics). At therapeutic plasma levels disopyramide shortens the sinus node recovery time, lengthens the effective refractory period of the atrium and has a minimal effect on the effective refractory period of the AV mode. However, because of the anticholinergic effects of some of the Type 1 antiarrhythmics, they should not be used in patients with glaucoma, myasthenia gravis or problems of urinary retention.

(b) Prior Art

As previously described, a number of compounds are useful in the treatment of arrhythmias. The pyridopyrimidinones of the instant invention are novel and represent a new class of compounds useful to treat arrhythmias with reduced anticholinergic activity.

GB—A—1 114 397 describes pyridopyrimidinone derivatives of the general formula

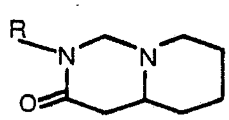

wherein R represents hydrogen or a lower alkyl group and their pharmaceutically acceptable salts, which exhibit pharmacological activities, especially analgesic, antiphlogistic, inflammation-inhibiting and antiallergic activity. The special compound of the above mentioned formula wherein R is methyl is also described in the Journal of the Chemical Society Perkin II, London, 1972, pages 1920—1925 without any pharmacological activities being mentioned.

SUMMARY OF THE INVENTION

It is an object of this invention to develop new chemical compounds with reduced anticholinergic activity that are useful in the treatment of arrhythmic conditions.

In accordance with this objective, it has been discovered that compounds of formula:

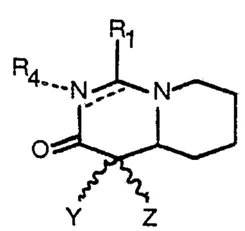

(IV)

wherein $R_1$ is:
a) hydrogen;
b) alkyl of from 1 to 6 carbon atoms, inclusive; or
c) phenyl;
wherein Y is:

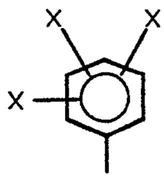

wherein each X is a substituent taken independently, selected from the group consisting of:
a) hydrogen;
b) halogen;
c) alkyl of from 1 to 6 carbon atoms, inclusive;
d) alkoxy of from 1 to 6 carbon atoms, inclusive; or
e) phenyl;
wherein Z is $-(CH_2)_n-NR_2R_3$, wherein n is 1 to 6 and wherein $R_2$ and $R_3$ are alkyl of from 1 to 6 carbon atoms, inclusive, $R_2$ and $R_3$ each being the same or different;
wherein $R_4$ is:
a) hydrogen; or
b) alkyl of 1 to 6 carbon atoms, inclusive;
and wherein the dotted line indicates an optional double bond, and when the dotted line represents a double bond, $R_4$ is absent;
and the pharmacologically acceptable salts are useful for their antiarrhythmic activity and exhibit reduced anticholinergic activity.

Examples of alkyl of from 1 to 6, inclusive, are methyl, ethyl, propyl, butyl, pentyl, hexyl and isomeric forms thereof.

Examples of halogen are chloro and bromo.

Examples of alkoxy of from 1 to 6 carbon atoms, inclusive are methoxy, ethoxy, propoxy, butoxy, pentoxy, hexoxy and isomeric forms thereof.

Antiarrhythmic activity was confirmed using ventricular arrhythmia induced in unanesthetized dogs. Ventricular arrhythmia is induced by a two-stage ligation of the anterior descending branch of the left coronary artery in each of two or more dogs. A test compound is rated active if it produces at least a 25% reduction in ectopic beats for a period of at least 10 minutes in half or more of the dogs tested. An active compound may act by suppressing the ectopic pacemaker, thus allowing normal sinus rhythm to return. This arrhythmia is considered similar in nature to the arrhythmia resulting from myocardial infarction in man. Quinidine and procainamide are active in this test and are effective in man.

By virtue of the antiarrhythmic activity, the compounds of Formula IV are useful in treating arrhythmias in mammals. A physician or veterinarian of ordinary skill could readily determine a subject who exhibits arrhythmias.

Regardless of the route of administration selected, the compounds of the present invention are formulated into pharmaceutically acceptable dosage forms by conventional methods known to those familiar with the pharmaceutical art.

The compounds can be administered in such oral unit dosage forms as tablets, capsules, pills, powders, or granules. They also may be administered intraperitoneally, subcutaneously, or intramuscularly, using forms known to those familiar with the pharmaceutical art. In general, the preferred form of administration is oral.

An effective but non-toxic quantity of the compounds is employed in treatment. The dosage regimen for preventing or treating arrhythmia by the compounds of this invention is selected in accordance with a variety of factors including the type, age, weight, sex, and medical condition of the patient, the severity of the arrhythmia, the route of administration and the particular compound employed. An ordinarily skilled physician or veterinarian will readily determine and prescribe the effective amount of the antiarrhythmic agent to prevent or arrest the progress of the condition. In so preceeding, the physician or veterinarian could employ relatively low dosages at first, subsequently increasing the dose until a maximum response is obtained.

Initial dosages of the compounds of the invention are ordinarily in the area of 1.0 mg/kg up to at least 10.0 mg/kg orally.

The compounds of this invention can also be administered as pharmacologically acceptable acid addition salts such as the hydrochloride, hydrobromide, hydroiodide, sulfate, phosphate, acetate, propionate, lactate, maleate, malate, succinate, tartrate and the like. Additionally, the compounds of this invention may be administered in a suitable hydrated form.

The compounds of this invention are prepared according to the general scheme on Chart A from the available racemic butanamides, I. Reduction of the pyridine ring of I, in which a new chiral center is formed, gives a mixture of four diastereomers, II. This may be accomplished by catalytic hydrogenation using catalysts such as Raney nickel, platinum, palladium or the like, or reaction with an alkali metal, such as sodium or lithium, in ammonia. The mixture of four compounds can be separated, for example by crystallization, into two pairs of racemates before further reactions are performed. In general, as can be expected for racemates, each racemic pair exhibits distinct and reproducible physical properties such as melting point and NMR spectra, and each racemate can be distinguished from the other by these and other properties. The intermediate compounds II are cyclized to corresponding diazabicyclic compounds III, for example by reaction with a dialkyl ketal or acetal of an amide, with an orthoester, or with other reactive formyl equivalents. Examples of amides which can be used as their ketals or acetals include dimethylformamide, dimethylacetamide, and dimethylbenzamide. Examples of orthoesters include triethylorthoformate, triethylorthoacetate, and triethylorthobenzoate. Examples of other reactive formyl equivalents incldue bis(dimethylamino)methoxymethane and tris(dimethylamino)methane. These bicyclic compounds can in turn be converted to compounds V by reductive procedures, for example catalytic hydrogenation, using Raney nickel, platinum, palladium or the like, or reaction with an active metal hydride such as lithium aluminum hydride, sodium borohydride, or one of the available derivative variations of these reagents. Compounds of Formula V can for example be prepared via the method in Example 24.

The following examples further illustrate details for the preparation and testing of the compounds and salts of the invention. The invention, which is fully set forth in the foregoing disclosure, is not to be construed as being limited either in spirit or scope by these examples. Those skilled in the art will readily understand that known variations of the conditions and processes of the following preparative procedures can also be used to prepare the compounds of the invention. All temperatures are in degrees celsius unless otherwise noted.

## DESCRIPTION OF THE PREFERRED EMBODIMENTS

### Example 1

Preparation of the intermediate 2-phenyl-2-(2-piperidinyl)-4-[N,N-bis(1-methylethyl)amino]butanamide, Racemate A(II).

A mixture of 105.3 g (0.31 mole) of racemic 2-phenyl-2-(2-pyridyl)-4-[N,N-bis(1-methylethyl)amino]-butanamide and 27 ml (ca. 0.32 mole) of concentrated aqueous hydrochloric acid in 2.5 liters of ethanol was hydrogenated ($3.45 \cdot 10^5$ Pa) over platinum oxide catalyst. After filtration to remove the catalyst, the reaction mixture was reduced to a syrupy residue, dissolved in an ice/water mixture, and neutralized with a slight excess of 25% sodium hydroxide. The product mixture was extracted into diethyl ether, which was dried over magnesium sulfate, filtered, and concentrated to dryness. The crude product was crystallized twice from Skellysolve B® containing a small amount of diethyl ether, to yield 26.0 g of the title compound as a white solid, m.p. 107—108°. Structure assignment was confirmed by proton and carbon-13 NMR spectra and by elemental analysis.

H—NMR ($CDCl_3$) δ ppm 0.83, 0.92, 7.33

### Example 2

Preparation of the intermediate 2-phenyl-2-(2-piperidinyl)-4-[N,N-bis(1-methylethyl)amino]butanamide, Racemate B(II).

The final filtrate from Example 1 was concentrated under a stream of nitrogen to an oil which slowly solidified. The crude material was chromatographed several times on silica gel to yield a total of 17 g of the title compound as a white solid, m.p. 81—82°. Structure assignment was confirmed by proton and carbon-13 NMR spectra and by elemental analysis.

H—NMR ($CDCl_3$) δ ppm 0.96, 1.05, 7.30

### Example 3

An alternative preparation of the intermediate 2-phenyl-2-(2-piperidinyl)-4-[N,N-bis(1-methylethyl)-amino]butanamide.

The compounds of Examples 1 and 2 were prepared by hydrogenation in the same manner as in Example 1, except that about 1.3 l of acetic acid was used as solvent and no hydrochloric acid was added.

### Example 4

Preparation of the claimed compound 5-phenyl-5-[2-[N,N-bis(1-methylethyl)amino]ethyl]-1,3-diazabicyclo[4.4.0]dec-2-en-4-one, Racemate A(III).

A solution of 16.35 g (47 mmole) of Racemate A(II) (of Example 1) and 8.9 g (60 mole) of dimethylformamide diethyl acetal in 100 ml of dimethylformamide was stirred overnight under nitrogen at room temperature. Water was added, and the resultant precipitate was collected, washed thoroughly with water,

and dried, yielding 12.9 g of the title compound as a white solid. Structure assignment was supported by NMR and by elemental analysis.

| | C | H | N | Characteristic Peaks |
|---|---|---|---|---|
| Calc'd | 74.33 | 9.36 | 11.82 | NMR ($CDCl_3$) δ ppm 0.77, 0.82, 0.85, 0.90, 7.18, 7.25 |
| Fd. | 74.27 | 9.51 | 11.91 | IR ($CHCl_3$) 2980, 1685, 1580 $cm^{-1}$, uV (MeOH), λmax 274—5 nm. |

Example 5

Preparation of the claimed compound 5-phenyl-5-[2-[N,N-bis(1-methylethyl)amino]ethyl]-1,3-diazabicyclo[4.4.0]decan-4-one, Racemate A(V).

A solution of 14.0 g (39.4 mmole) of Racemate A(III), prepared by the method of Example 4, in 260 ml of ethanol was hydrogenated (0.345 bar) over 5% palladium on carbon. After the catalyst was removed by filtration, the solvent was removed from the filtrate to give a white solid. Recrystallization from ethyl acetate afforded 10.0 g of the title compound, m.p. 204—206°. Structure assignment was confirmed by proton and carbon-13 NMR and infrared spectra and by elemental analysis.

| | C | H | N | |
|---|---|---|---|---|
| Calc'd | 73.91 | 9.87 | 11.75 | NMR ($CDCl_3$) δ ppm 1.03, 1.06, 1.12, 1.15, 7.15 |
| Fd | 73.90 | 9.71 | 11.83 | IR ($CHCl_3$) 3410, 2985, 1680 $cm^{-1}$ |

Example 6

Preparation of the claimed compound 5-phenyl-5-[2-[N,N-bis(1-methylethyl)amino]ethyl]-1,3-diazabicyclo[4.4.0]dec-2-en-4-one, Racemate B(III).

The title compound was prepared by the method of Example 4 using 1.7 g (4.9 mmole) of Racemate B(II) (of Example 2) and 1.0 g of dimethylformamide diethyl acetal in 12 ml of dimethylformamide. After one day the solution was concentrated to dryness *in vacuo.* The residue was recrystallized from diethyl ether to give the title compound as a white solid. Structure assignment was confirmed by proton NMR, ultraviolet, and infrared spectra and by elemental analysis, M.P. 155°—158°C.

| | C | H | N | NMR ($CDCl_3$) δ ppm 0.80, 0.82, 0;.89, 0.91, 7.29 |
|---|---|---|---|---|
| Calc'd. | 74.33 | 9.36 | 11.82 | uV (MeoH) λmax 273—274 nm |
| Fd. | 74.22 | 9.53 | 11.84 | IR ($CHCl_3$) 2980, 1680, 1585 $cm^{-1}$. |

Example 7

Preparation of claimed compound 5-phenyl-5-[2-[N,N-bis(1-methylethyl)amino]ethyl]-1,3-diazabicyclo-[4.4.0]decan-4-one, Racemate B(V).

The title compound was prepared by the method of Example 5 using 200 mg (0.56 mmole) of Racemate B(III) (of Example 6). The reaction mixture was concentrated to dryness and the residue was triturated with diethyl ether. The structure of the product, a white solid (0.18 g), was confirmed by proton NMR, infrared, and ultraviolet spectra and by elemental analysis, M.P. 164—166°C.

| | C | H | N | |
|---|---|---|---|---|
| Calc'd | 73.91 | 9.87 | 11.75 | NMR ($CDCl_3$) δ ppm 1.00, 1.10, 7.05, 7.55 |
| Fd. | 74.00 | 9.85 | 11.65 | |

Example 8

Preparation of the claimed compound 2-methyl-5-phenyl-5-[2-[N,N-bis(1-methylethylamino]ethyl]-1,3-diazabicyclo[4.4.0]dec-2-en-4-one, Racemate A(III, $R_1$=methyl).

A mixture of 20.0 g (57.0 mmole) of Racemate A(II) (of Example 1) and 22.0 g (165 mmole) of N,N-dimethylacetamide dimethyl acetal was heated at 80° for about 14 hours. After cooling, the reaction mixture was diluted with diethyl ether, filtered, washed with diethyl ether, and air-dried, giving 6.0 g of the title compound, m.p. 191—192°. The product had the expected elemental composition.

| | C | H | N | |
|---|---|---|---|---|
| Calc'd. | 74.75 | 9.55 | 11.37 | NMR ($CDCl_3$) δ ppm 0.77, 0.80, 0.87, 0.90, 1.95, m 7.23 |
| Fd | 74.49 | 9.57 | 11.39 | IR ($CHCl_3$) 2970, 1675, 1540, 1480 $cm^{-1}$, uV (MeOH), λmax 273.274 nm. |

Example 9

Preparation of the claimed compound 2-methyl-5-phenyl-5-[2-N,N-bis(1-methylethyl)amino]ethyl]-1,3-diazabicyclo[4.4.0]dec-2-en-4-one, Racemate B(III, $R_1$=methyl).

The title racemate is prepared by the method of Example 8 using Racemate B(II) (of Example 2).

Example 10

Preparation of the claimed compound 2-methyl-5-phenyl-5-[2-[N,N-bis(1-methylethyl)amino]ethyl]-1,3-diazabicyclo[4.4.0]decan-4-one, Racemate A(V, $R_1$=methyl).

To a solution of 2.8 g (7.6 mmole) of Racemate A(III, $R_1$=methyl) (of Example 8) in 40 ml of freshly distilled tetrahydrofuran was added, in portions, 0.57 g (15.0 mmole) of lithium aluminium hydride. The mixture was stirred at ambient temperature for two hours and then cooled in an ice bath. The reaction was quenched by adding 1.2 ml of water, 1.2 ml of 15% aqueous sodium hydroxide, and another 3.0 ml of water, and the resulting precipitate was removed by filtration. The filtrate was concentrated to dryness, and the residue dried in vacuo at about 50° for 12 to 15 hours, giving 2.4 g of the title compound as a white solid. Structure assignment was confirmed by proton NMR and infrared spectra and by elemental analysis, M.P. 70°—75°C.

| | C | H | N | |
|---|---|---|---|---|
| Calc'd. | 74.35 | 10.04 | 11.31 | NMR ($CDCl_3$) δ ppm 0.99, 1.01, 1.08, 1.10, 1.28, 1.35, 7.50 |
| Fd. | 74.36 | 9.95 | 11.34 | IR ($CHCl_3$) 3400, 2985, 1665 $cm^{-1}$ |

Example 11

Preparation of the claimed compound 2-methyl-5-phenyl-5-[2-[N,N-bis(1-methylethyl)amino]ethyl]-1,3-diazabicyclo[4.4.0]decan-4-one, Racemate B(V, $R_1$=methyl).

The title racemate is prepared by the method of Example 10 using Racemate B(III) (of Example 9).

Example 12

Preparation of the intermediate racemic 2-(4-phenylphenyl)-2-(2-pyridyl)-4-[N,N-bis(1-methylethyl)-amino]butanenitrile.

A mixture of 50.0 g (0.16 mole) of 2-(4-phenylphenyl)-4-[N,N-bis(1-methylethyl)amino]butanenitrile and 29.7 g of potassium hydride (about 0.26 mole as a 35% dispersion in oil) in 1000 ml of toluene was heated at 65°. A solution of 32 g (0.20 mole) of 2-bromopyridine in 450 ml of toluene was added slowly. After addition was complete, the mixture was stirred an additional 30 minutes, cooled in an ice bath, and treated with 750 ml of water. The toluene layer was separated and extracted with 10% aqueous hydrochloric acid. The acidic aqueous layer was neutralized with a slight excess of aqueous sodium hydroxide and extracted with dichloromethane. The organic phase was washed with brine, dried over magnesium sulfate, filtered, and concentrated to dryness, giving 56.5 g of the title compound as an oil having the expected NMR spectrum. The intermediate nitrile was used without further purification.

Example 13

Preparation of the intermediate racemic 2-(4-phenylphenyl)-2-(2-pyridyl)-4-[(N,N-bis(1-methylethyl)-amino]butanamide.

A mixture of 1.0 g (2.5 mmole) of the nitrile from Example 12 and 2 g of powdered potassium hydroxide in 10 ml of *t*-butyl alcohol was heated at reflux for about 12 hours. After cooling, the reaction mixture was treated with water and extracted with diethyl ether. The organic phase was dried over magnesium sulfate, filtered, and concentrated to an oil. Crystallization from Skellysolve B® afforded the title compound (0.46 g) as a white solid. Structure assignment was supported by proton NMR and infrared spectra and by elemental analysis.

| | C | H | N | |
|---|---|---|---|---|
| | | | | NMR ($CDCl_3$) δ ppm 0.88, 0.95, 7.0—7.75, 8.50, 8.55 |
| Calc'd. | 78.04 | 8.00 | 10.11 | IR ($CHCl_3$) 3350, 2970, 1670 $cm^{-1}$ |
| Fd. | 77.92 | 8.06 | 9.88 | |

Example 14

Preparation of the claimed compound 5-(4-phenylphenyl)-5-[2-[N,N-bis(1-methylethyl)amino]ethyl]-1,3-diazabicyclo[4.4.0]dec-2-en-4-one, Racemtate C(III, X=phenyl).

The title racemate, m.p. 257—258°, was prepared by the methods of Examples 1 and 4 using the

racemic amide of Example 13. Structure assignment was supported by NMR and infrared spectra and by elemental analysis.

| | C | H | N | |
|---|---|---|---|---|
| Calc'd. | 77.92 | 8.64 | 9.73 | NMR ($CDCl_3$) δ ppm 0.79, 0.85, 0.88, 0.94, 7.05—7.65 |
| Fd. | 77.93 | 8.79 | 9.74 | IR ($CHCl_3$) 2985, 1680, 1575 $cm^{-1}$ |

Example 15

Preparation of the claimed compound 5-(4-phenylphenyl)-5-[2-[N,N-bis(1-methylethyl)amino]ethyl]-1,3-diazabicyclo[4.4.0]dec-2-en-4-one, Racemtate D(III, X=phenyl).

The title racemate is prepared by the methods of Examples 2 and 4 using the racemic amide of Example 13.

Example 16

Preparation of the claimed compound 5-(4-phenylphenyl)-5-[2-[N,N-bis(1-methylethyl)amino]ethyl]-1,3-diazabicyclo[4.4.0]decan-4-one, Racemate C(V, X=phenyl).

The title racemate was prepared by the method of Example 5 using Racemate C(III, X=phenyl) (of Example 14). Structure assignment was supported by the NMR, infrared, and ultraviolet spectra and by elemental analysis.

| | C | H | N | |
|---|---|---|---|---|
| Calc'd. | 77.56 | 9.06 | 9.65 | NMR ($CDCl_3$) δ ppm 1.02, 1.05, 1.10, 1.13, 7.05—7.65 |
| Fd | 77.11 | 9.03 | 9.74 | IR ($CHCl_3$) 3400, 2960, 1660 $cm^{-1}$, uV (MeOH), λmax 254 nm |

Example 17

Preparation of the claimed compound 5-(4-phenylphenyl-5-[2-[N,N-bis(1-methylethyl)amino]ethyl]-1,3-diazabicyclo[4.4.0]dec-4-one, Racemate D(V, X=phenyl).

The title racemate is prepared by the method of Example 5 using Racemate D(III, X=phenyl) (of Example 15).

Example 18

Preparation of the claimed compound 5-(2-chlorphenyl)-5-[2-[N,N-bis(1-methylethyl)amino]ethyl]-1,3-diazabicyclo[4.4./0]dec-2-en-4-one, Racemtate E(III, X=chloro).

The title racemate, m.p. 154—156°, was prepared by the methods of Examples 1 and 4 using 28.5 g (76 mmole) of racemic 2-(2-chlorophenyl)-2-(2-pyridyl)-4-[N,N-bis(1-methylethyl)amino]butanamide.

| | C | H | N | Cl | |
|---|---|---|---|---|---|
| Calc'd. | 67.76 | 8.27 | 10.78 | 9.09 | NMR ($CDCl_3$) δ ppm 0.90, 0.95, 0.98, 1.03, 7.05—7.45 |
| Fd. | 67.90 | 8.28 | 10.54 | 9.10 | IR ($CHCl_3$) 2960, 1680, 1580 $cm^{-1}$, uV (MeOH) λmax 273—4 nm |

Example 19

Preparation of the claimed compound 5-(2-chlorophenyl)-5-[N,N-bis(1-methuylethyl)amino]ethyl]-1,3-diazabicyclo[4.4.0]dec-2-en-4-one, Racemate F(III, X=chloro).

The title racemate is prepared by the methods of Examples 2 and 4 using racemic 2-(2-chlorophenyl)-2-(2-pyridyl)-4-[N,N-bis(1-methylethyl)amino]butanamide.

Example 20

Preparation of the claimed compound 5-(2-chlorophenyl)-5-[N,N-bis(1-methylethyl)amino]ethyl]-1,3-diazabicyclo[4.4.0]decan-4-one, Racemate E(V, X=chloro).

The title racemate, m.p. 232—235°, was prepared by the method of Example 10 using Racemate E(III, X=chloro) (of Example 18). Structure assignment was supported by NMR, infrared, and ultraviolet spectra and by elemental analysis.

| | C | H | N | Cl | |
|---|---|---|---|---|---|
| Calc'd. | 67.41 | 8.74 | 10.72 | 9.04 | NMR ($CDCl_3$) δ ppm 1.01, 1.09, 7.05—7.65 |
| Fd. | 67.46 | 8.93 | 10.40 | 8.76 | IR ($CHCl_3$) 3410, 2970, 1670 $cm^{-1}$ |

Example 21

Preparation of the claimed compound 5-(2-chlorophenyl)-5-[2-[N,N-bis(1-methylethyl)amino]-ethyl]-1,3-diazabicyclo[4.4.0]decan-4-one, Racemate F(V, X=chloro).

The title racemate is prepared by the method of Example 10 using Racemate F(III, X=chloro) (of Example 19).

Example 22

Table 1: Preparation of Substituted 1,3-Diazabicyclo [4.4.0]dec-2-en-4-ones (claimed compounds)

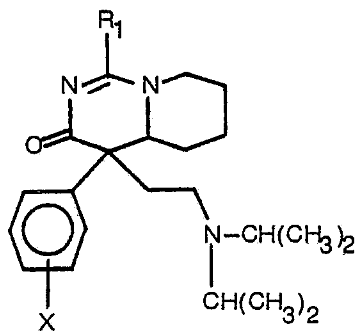

| X | $R_1$ | Racemate | Examples for Methods Used* | Example for Amide Precursor (II) |
|---|---|---|---|---|
| H | Phenyl | G | 4 or 8 | 1 |
| H | Phenyl | H | 6 or 9 | 2 |
| 4-Phenyl | Methyl | K | 4 or 8 | 13 |
| 4-Phenyl | Methyl | L | 6 or 9 | 13 |
| 4-Phenyl | Phenyl | M | 4 or 8 | 13 |
| 4-Phenyl | Phenyl | N | 6 or 9 | 13 |
| 2-Chloro | Methyl | P | 4 or 8 | # |
| 2-Chloro | Methyl | Q | 6 or 9 | # |
| 2-Chloro | Phenyl | R | 4 or 8 | # |
| 2-Chloro | Phenyl | S | 6 or 9 | # |

* All methods require use of dialkyl ketal of appropriate amide $R_1$—CON(Alkyl)$_2$. Variations on the syntheses use similar orthoesters or active methylene compounds of general type $R_1$—$CX_3$, where X = OAlkyl, N(Alkyl)$_2$ (or combinations thereof), and the like.

# Precursor: 2-(2-chlorophenyl)-2-(2-pyridyl)-4-[N,N-bis (1-methylethyl)amino]butanamide.

Example 23

Table 2: Preparation of Substituted 1,3-Diazabicyclo[4.4.0]decan-4-ones (claimed compounds) from compounds of Table 1.

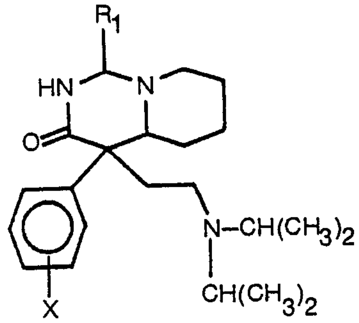

| X | $R_1$ | Racemate | Examples for Methods used* |
|---|---|---|---|
| H | Phenyl | G | 5 or 10 |
| H | Phenyl | H | 7 or 11 |
| 4-Phenyl | Methyl | K | 5 or 10 |
| 4-Phenyl | Methyl | L | 7 or 11 |
| 4-Phenyl | Phenyl | M | 5 or 10 |
| 4-Phenyl | Phenyl | N | 7 or 11 |
| 2-Chloro | Methyl | P | 5 or 10 |
| 2-Chloro | Methyl | Q | 7 or 11 |
| 2-Chloro | Phenyl | R | 5 or 10 |
| 2-Chloro | Phenyl | S | 7 or 11 |

Example 24

Preparation of the claimed compound:

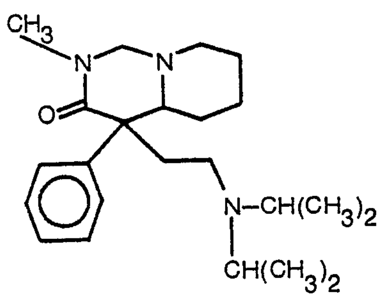

To 0.26 g (5.5 mmoles) of 50% sodium hydride mineral oil dispersion, previously washed with Skellysolve B® to remove the mineral oil, in 25 ml of DMF was added 1.63g (4.6 mmoles) of Racemate A of Example 5 and the reaction mixture was stirred at room temperature for 15 minutes. After this time 0.97 g (6.8 mmoles) of methyl iodide was added to the reaction mixture and it was stirred for an additional 40 min. The excess sodium hydride was then destroyed with $H_2O$ before partitioning the reaction mixture between $H_2O$ and ether/ethylacetate (1:1). The organic phase was extracted 3 times with 1N HCl solution and the acidic solution was backwashed once with ether. The aqueous acidic solution was basified with 5% sodium hydroxide solution and extracted with ether. The ether extracts were dried over sodium sulfate. Solvent

removal gave an oil which upon chromatography over silica gel using $CHCl_3/EtOH/NH_4OH$ as the eluent afforded pure title compound of the above structure as an oil.

| | C | H | N |
|---|---|---|---|
| Calc'd | 74.35 | 10.04 | 11.31 |
| Found | 74.35 | 10.22 | 10.80 |

NMR($CDCL_3$) δ ppm 1.00, 1.10, 3.03, 7.17—7.33
IR($CHCL_3$) 1650 $cm^{-1}$

Example 25

Preparation of the claimed compound

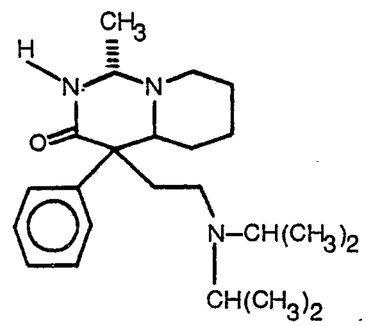

To 2.29 g (12 mmoles) of cuprous iodide in 20 ml of dry tetrahydrafuran (THF) cooled to *ca.* −20°C in an argon atmosphere was added dropwise 8.3 ml of 2.9M methyl magnesium chloride/THF (24 mmoles). After stirring for 10 minutes, 2.84 g (8 mmoles) of Racemate A of Example 4 in 60 ml of THF was added dropwise at the above temperature. After addition the reaction mixture was stirred for an additional hour and then allowed to warm to room temperature where it was also stirred for one hour. After cooling to 0°C in HCl solution was added until the solution attained a pH of 3. After stirring for 30 minutes the solution was basified with a potassium hydroxide solution to pH 12 and the two layers were separated. The aqueous phase was extracted with additional portions of ether and the combined extracts were washed three times with 3% $NH_4OH$ solution followed by saturated sodium chloride solution. The solution was then dried over sodium sulfate and upon solvent removal a pale yellow solid remained. Chromatography of this residue over silica gel using $CH_2Cl_2/EtOH/NH_4OH$(85:14:1) as an eluent afforded the pure compound melting at about 142—145°C of the above structure.

| | C | H | N |
|---|---|---|---|
| Calc'd | 74.35 | 10.04 | 11.31 |
| Found | 73.97 | 10.05 | 11.22 |

NMR($CDCl_3$) δ ppm 0.85, 0.87, 0.94, 0.96, 1.08, 1.14.
IR($CHCl_3$) 3400, 1665 $cm^{-1}$.

CHART A

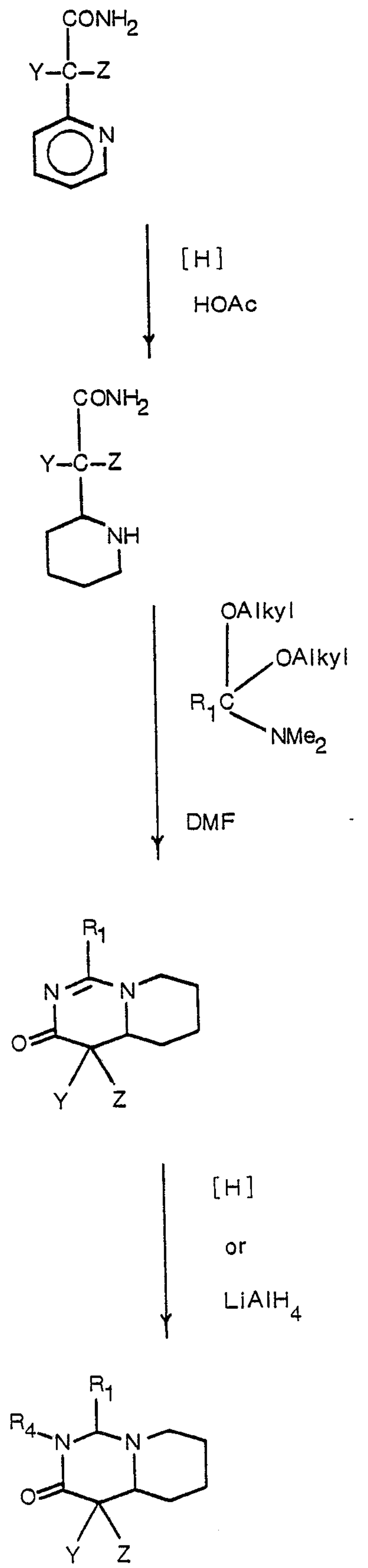


I

II

III

V

**Claims**

1. A compound of the formula:

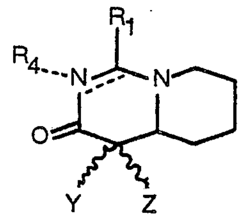

(IV)

wherein $R_1$ is
a) hydrogen;
b) alkyl of from 1 to 6 carbon atoms, inclusive; or
c) phenyl;
wherein Y is

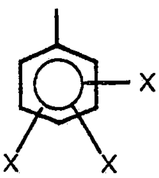

wherein each X is a substituent taken independently, selected from the group consisting of:
a) hydrogen;
b) halogen;
c) alkyl of from 1 to 6 carbon atoms, inclusive;
d) alkoxy of from 1 to 6 carbon atoms, inclusive; or
e) phenyl;
wherein Z is $-(CH_2)_n-NR_2R_3$, wherein n is 1 to 6 and wherein $R_2$ and $R_3$ are alkyl of from 1 to 6 carbon atoms, inclusive, $R_2$ and $R_3$ each being the same or different; wherein $R_4$ is:
a) hydrogen; or
b) alkyl of 1 to 6 carbon atoms, inclusive;
and wherein the dotted line indicates an optional double bond, and when the dotted line represents a double bond, $R_4$ is absent;
and the pharmacologically acceptable salts.

2. A compound according to Claim 1 wherein $R_1$ is hydrogen.

3. 5 - phenyl - 5 - [2 - [N,N - bis(1 - methylethyl)amino]ethyl] - 1,3 - diazabicylo[4.4.0.]dec - 2 - en - 4 - one, and the corresponding isomers and racemates thereof, compounds according to Claim 2.

4. 5 - phenyl - 5 - [2 - [N,N - bis(1 - methylethyl)amino]ethyl] - 1,3 - diazabicycylo[4.4.0]decan - 4 - one, and the corresponding isomers and racemates thereof, compounds according to Claim 2.

5. 5 - (4 - phenylphenyl) - 5 - [2 - [N,N - bis(1 - methylethyl)amino]ethyl] - 1,3-diazabicyclo[4.4.0]dec - 2 - en - 4 - one, and the corresponding isomers and racemates thereof, compounds according to Claim 2.

6. 5 - (4 - phenylphenyl) - 5 - [2 - [N,N - bis(1 - methylethyl)amino]ethyl] - 1,3-diazabicyclo[4.4.0]decan - 4 - one, and the corresponding isomers and racemates thereof, compounds according to Claim 2.

7. 5 - (2 - chlorophenyl) - 5 - [2 - [N,N - bis(1 - methylethyl)amino]ethyl] - 1,3-diazabicyclo[4.4.0]dec - 2 - en - 4 - one, and the corresponding isomers and racemates thereof, compounds according to Claim 2.

8. 5 - (2 - chlorophenyl) - 5 - [2 - [N,N - bis(1 - methylethyl)amino]ethyl] - 1,3-diazabicyclo[4.4.0]decan - 4 - one, and the corresponding isomers and racemates thereof, compounds according to Claim 2.

9. A compound according to Claim 1 wherein $R_1$ is alkyl of from 1 to 6 carbon atoms, inclusive.

10. 2 - methyl - 5 - phenyl - 5 - [2 - [N,N - bis(1 - methylethylamino]ethyl] - 1,3-diazabicyclo[4.4.0]dec - 2 - en - 4 - one, and the corresponding isomers and racemates thereof, compounds according to Claim 9.

11. 2 - methyl - 5 - phenyl - 5 - [2 - [N,N - bis(1 - methylethyl)amino]ethyl] - 1,3-diazabicyclo[4.4.0]decan - 4 - one, and the corresponding isomers and racemates thereof, compounds according to Claim 9.

12. 2 - methyl - 5 - (4 - phenylphenyl) - 5 - [2 - [N,N - bis(1 - methylethyl)amino]ethyl] - 1,3-diazabicyclo[4.4.0]dec - 2 - en - 4 - one, and the corresponding isomers and racemates thereof, compounds according to Claim 9.

13. 2 - methyl - 5 - (4 - phenylphenyl) - 5 - [2 - [N,N - bis(1 - methylethyl)amino]ethyl] - 1,3-diazabicyclo[4.4.0]decan - 4 - one, and the corresponding isomers and racemates thereof, compounds according to Claim 9.

14. 2 - methyl - 5 - (2 - chlorophenyl) - 5 - [2 - [N,N - bis(1 - methylethyl)amino]ethyl] - 1,3-diazabicyclo[4.4.0]dec - 2 - en - 4 - one, and the corresponding isomers and racemates thereof, compounds according to Claim 9.

15. 2 - methyl - 5 - (2 - chlorophenyl) - 5 - [2 - [N,N - bis(1 - methylethyl)amino]ethyl] - 1,3-diazabicyclo[4.4.0]decan - 4 - one, and the corresponding isomers and racemates thereof, compounds according to Claim 9.

16. A compound according to claim 1 wherein $R_1$ is phenyl.

17. 2 - phenyl - 5 - phenyl - 5 - [2 - [N,N - bis(1 - methylethyl)amino]ethyl] - 1,3-diazabicyclo[4.4.0]dec - 2 - en - 4 - one, and the corresponding isomers and racemates thereof, compounds according to claim 16.

18. 2 - phenyl - 5 - phenyl - 5 - [2 - [N,N - bis(1 - methylethyl)amino]ethyl] - 1,3-diazabicyclo[4.4.0]decan - 4 - one, and the corresponding isomers and racemates thereof, compounds according to claim 16.

19. 2 - phenyl - 5 - (4 - phenylphenyl) - 5 - [2 - [N,N - bis(1 - methylethyl)amino]ethyl] - 1,3-diazabicyclo[4.4.0]dec - 2 - en - 4 - one, and the corresponding isomers and racemates thereof, compounds according to claim 16.

20. 2 - phenyl - 5 - (4 - phenylphenyl) - 5 - [2 - [N,N - bis(1 - methylethyl)amino]ethyl] - 1,3-diazabicyclo[4.4.0]decan - 4 - one, and the corresponding isomers and racemates thereof, compounds according to claim 16.

21. 2 - phenyl - 5 - (2 - chlorophenyl) - 5 - [2 - [N,N - bis(1 - methylethyl)amino]ethyl] - 1,3-diazabicyclo[4.4.0]dec - 2 - en - 4 - one, and the corresponding isomers and racemates thereof, compounds according to claim 16.

22. 2 - phenyl - 5 - (2 - chlorophenyl) - 5 - [2 - [N,N - bis(1 - methylethyl)amino]ethyl] - 1,3-diazabicyclo[4.4.0]decan - 4 - one, and the corresponding isomers and racemates thereof, compounds according to claim 16.

**Patentansprüche**

1. Verbindung der Formel:

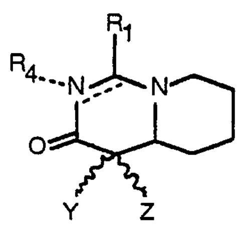

(IV)

worin $R_1$

a) Wasserstoff
b) eine Alkylgruppe mit 1 bis 6 Kohlenstoffatomen oder
c) eine Phenylgruppe bedeutet;

worin Y

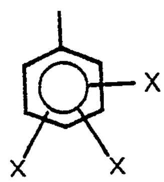

ist;

worin jedes X ein voneinander unabhängiger Substituent ist, ausgewählt aus der nachhfolgenden Gruppe:

a) Wasserstoff
b) Halogen,
c) eine Alkylgruppe mit 1 bis 6 Kohlenstoffatomen,
d) eine Alkoxygruppe mit 1 bis 6 Kohlenstoffatomen, oder
e) eine Phenylgruppe;

worin $Z—(CH_2)_n—NR_2R_3$ ist, worin n die Zahl 1 bis 6 bedeutet und worin $R_2$ und $R_3$ Alkylgruppen mit 1 bis 6 Kohlenstoffatomen bedeuten, wobei $R_2$ und $R_3$ gleich oder verschieden sind;

worin $R_4$

a) Wasserstoff oder
b) eine Alkylgruppe mit 1 bis 6 Kohlenstoffatomen ist

und worin die punktierte Linie eine wahlweise Doppelbindung anzeigt und wenn die punktierte Linie eine Doppelbindung darstellt, $R_4$ abwesend ist;
und die pharmazeutisch akzeptablen Salze.

2. Verbindung gemäß Anspruch 1, worin $R_1$ Wasserstoff ist.

3. 5 - Phenyl - 5 - [2 - [N,N - bis(1 - methylethyl)amino]ethyl] - 1,3 - diazabicyclo[4.4.0]dec - 2 - en - 4 - one, und die entsprechenden Isomere und Racemate, Verbindungen gemäß Anspruch 2.

4. 5 - Phenyl - 5 - [2 - [N,N - bis(1 - methylethyl)amino]ethyl] - 1,3 - diazabicyclo[4.4.0]decan - 4 - one, und die entsprechenden Isomere und Racemate, Verbindungen gemäß Anspruch 2.

5. 5 - (4 - Phenylphenyl) - 5 - [2 - [N,N - bis(1 - methylethyl)amino]ethyl] - 1,3 - diazabicyclo[4.4.0]-dec - 2 - en - 4 - one, und die entsprechenden Isomere und Racemate, Verbindungen entsprechend Anspruch 2.

6. 5 - (4 - Phenylphenyl) - 5 - [2 - [N,N - bis(1 - methylethyl)amino]ethyl] - 1,3 - diazabicyclo[4.4.0]-decan - 4 - one, und die entsprechenden Isomere und Racemate, Verbindungen entsprechend Anspruch 2.

7. 5 - (2 - Chlorphenyl) - 5 - [2 - [N,N - bis(1 - methylethyl)amino]ethyl] - 1,3 - diazabicyclo[4.4.0]-dec - 2 - en - 4 - one, und die entsprechenden Isomere und Racemate, Verbindungen entsprechend Anspruch 2.

8. 5 - (2 - Chlorphenyl) - 5 - [2 - [N,N - bis(1 - methylethyl)amino]ethyl] - 1,3 - diazabicyclo[4.4.0]-decan - 2 - en - 4 - one, und die entsprechenden Isomere und Racemate, Verbindungen entsprechend Anspruch 2.

9. Verbindung gemäß Anspruch 1, worin $R_1$ eine Alkylgruppe mit 1 bis 6 Kohlenstoffatomen bedeutet.

10. 2 - Methyl - 5 - phenyl - 5 - [2 - [N,N - bis(1 - methylethyl)amino]ethyl] - 1,3 - diazabicyclo-[4.4.0]dec - 2 - en - 4 - one, und die entsprechenden Isomere und Racemate, Verbindungen gemäß Anspruch 9.

11. 2 - Methyl - 5 - phenyl - 5 - [2 - [N,N - bis(1 - methylethyl)amino]ethyl] - 1,3 - diazabicyclo-[4.4.0]decan - 4 - one, und die entsprechenden Isomere und Racemate, Verbindungen gemäß Anspruch 9.

12. 2 - Methyl - 5 - (4 - phenylphenyl) - 5 - [2 - [N,N - bis(1 - methylethyl)amino]ethyl] - 1,3 - diaza-bicyclo[4.4.0]dec - 2 - en - 4 - one, und die entsprechenden Isomere und Racemate, Verbindungen gemäß Anspruch 9.

13. 2 - Methyl - 5 - (4 - phenylphenyl) - 5 - [2 - [N,N - bis(1 - methylethyl)amino]ethyl] - 1,3 - diaza-bicyclo[4.4.0]decan - 4 - one, und die entsprechenden Isomere und Racemate, Verbindungen gemäß Anspruch 9.

14. 2 - Methyl - 5 - (2 - chlorphenyl - 5 - [2 - [N,N - bis(1 - methylethyl)amino]ethyl] - 1,3 - diaza-bicyclo[4.4.0]dec - 2 - en - 4 - one, und die entsprechenden Isomere und Racemate, Verbindungen gemäß Anspruch 9.

15. 2 - Methyl - 5 - (2 - chlorphenyl - 5 - [2 - [N,N - bis(1 - methylethyl)amino]ethyl] - 1,3 - diaza-bicyclo[4.4.0]decan - 4 - one, und die entsprechenden Isomere und Racemate, Verbindungen gemäß Anspruch 9.

16. Verbindung gemäß Anspruch 1, worin $R_1$ eine Phenylgruppe bedeutet.

17. 2 - Phenyl - 5 - phenyl - 5 - [2 - [N,N - bis(1 - methylethyl)amino]ethyl] - 1,3 - diazabicyclo-[4.4.0]dec - 2 - en - 4 - one, und die entsprechenden Isomere und Racemate, Verbindungen gemäß Anspruch 16.

18. 2 - Phenyl - 5 - phenyl - 5 - [2 - [N,N - bis(1 - methylethyl)amino]ethyl] - 1,3 - diazabicyclo-[4.4.0]decan - 4 - one, und die entsprechenden Isomere und Racemate, Verbindungen gemäß Anspruch 16.

19. 2 - Phenyl - 5 - (4 - phenylphenyl) - 5 - [2 - [N,N - bis(1 - methylethyl)amino]ethyl] - 1,3 - diaza-bicyclo[4.4.0]dec - 2 - en - 4 - one, und die entsprechenden Isomere und Racemate, Verbindungen gemäß Anspruch 16.

20. 2 - Phenyl - 5 - (4 - phenylphenyl) - 5 - [2 - [N,N - bis(1 - methylethyl)amino]ethyl] - 1,3 - diaza-bicyclo[4.4.0]decan - 4 - one, und die entsprechenden Isomere und Racemate, Verbindungen gemäß Anspruch 16.

21. 2 - Phenyl - 5 - (2 - chlorphenyl) - 5 - [2 - [N,N - bis(1 - methylethyl)amino]ethyl] - 1,3 - diaza-bicyclo[4.4.0]dec - 2 - en - 4 - one, und die entsprechenden Isomere und Racemate, Verbindungen gemäß Anspruch 16.

22. 2 - Phenyl - 5 - (2 - chlorphenyl) - 5 - [2 - [N,N - bis(1 - methylethyl)amino]ethyl] - 1,3 - diaza-bicyclo[4.4.0]decan - 2 - en - 4 - one, und die entsprechenden Isomere und Racemate, Verbindungen gemäß Anspruch 16.

**Revendications**

1. Une composé de formule:

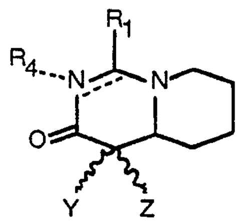

(IV)

dans laquelle $R_1$ est:

a) l'hydrogène;

b) alkyle en $C_1$—$C_6$ inclus; ou

c) phényle;

dans laquelle Y est:

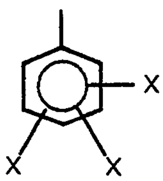

dans laquelle chaque X est un substituant choisi indépendamment parmi:

a) l'hydrogène;

b) un halogène;

c) alkyle en $C_1$—$C_6$ inclus;

d) alcoxy en $C_1$—$C_6$ inclus; ou

e) phényle;

dans laquelle Z est —$(CH_2)_n$—$NR_2R_3$ où n est un nombre de 1 à 6 et où $R_2$ et $R_3$ sont identiques ou différents et représentent chacun alkyle en $C_1$—$C_6$ inclus;

dans laquelle $R_4$ est:

a) l'hydrogène; ou

b) alkyle en $C_1$—$C_6$ inclus;

et dans laquelle le trait interrompu indique une double liaison facultative et, lorsque le trait interrompu représente une double liaison, $R_4$ est absent;

est les sels acceptables en pharmacie.

2. Une composé selon la revendication 1, dans lequel $R_1$ est l'hydrogène.

3. La 5 - phényl - 5 - [2 - [N,N - bis(1 - méthyléthyl)amino] - ethyl - 1,3 - diazabicyclo[4.4.0]déc - 2 - ène - 4 - one, et ses isomères et racémates correspondants, composés selon la revendication 2.

4. La 5 - phényl - 5 - [2 - [N,N - bis(1 - méthyléthyl)amino] - ethyl - 1,3 - diazabicyclo[4.4.0]décane - 2 - ène - 4 - one, et ses isomères et racémates correspondants, composés selon la revendication 2.

5. La 5 - (4 - phénylphényl) - 5 - [2 - [N,N - bis(1-méthyléthyl) - amino]-éthyl]1,3 - diazabicyclo[4.4.0]déc - 2 - ène - 4 - one, et ses isomères et racémates correspondants, composés selon la revendication 2.

6. La 5 - (4 - phénylphényl) - 5 - [2 - [N,N - bis(1 - méthyléthyl) - amino]-éthyl]-1,3 - diazabicyclo[4.4.0]décane - 4 - one, et ses isomères et racémates correspondants, composés selon la revendication 2.

7. La 5 - (2 - chlorophenyl) - 5 - [2 - [N,N - bis(1-méthyléthyl) - amino]-éthyl]-1,3 - diazabicyclo[4.4.0]déc - 2 - éne - 4 - one, et ses isomères et racémates correspondants, composés selon la revendication 2.

8. La 5 - (2 - chlorophényl) - 5 - [2 - [N,N - bis(1 - méthyléthyl) - amino]-éthyl]-1,3 - diazabicyclo[4.4.0]décane - 4 - one, et ses isomères et racémates correspondants, composés selon la revendication 2.

9. Un composé selon la revendication 1, dans lequel $R_1$ est alkyle en $C_1$—$C_6$ inclus.

10. La 2 - méthyl - 5 - phényl - 5 - [2 - [N,N - bis(1-méthyléthyl) - amino]-éthyl]-1,3 - diazabicyclo[4.4.0]déc - 2 - ène - 4 - one, et ses isomères et racémates correspondants, composés selon la revendication 9.

11. La 2 - méthyl - 5 - phényl - 5 - [2 - [N,N - bis(1-méthyléthyl) - amino]éthyl]-1,3 - diazabicyclo[4.4.0]décane - 4 - one, et ses isomères et racémates correspondants, composés selon la revendication 9.

12. La 2 - méthyl - 5 - (4 - phénylphényl) - 5 - [2 - [N,N - bis(1 - méthyl - éthyl)amino]éthyl] - 1,3 - diazabicyclo[4.4.0]déc - 2 - ène - 4 - one, et ses isomères et racémates correspondants, composés selon la revendication 9.

13. La 2 - méthyl - 5 - (4 - phénylphényl) - 5 - [2 - [N,N - bis(1 - méthyl - éthyl)amino]éthyl]- 1,3 - diazabicyclo[4.4.0]décane - 4 - one, et ses isomères et racémates correspondants, composés selon la revendication 9.

14. La 2 - méthyl - 5 - (2 - chlorophényl) - 5 - [2- [N,N - bis(1 - méthyl - éthyl)amino]éthyl] - 1,3 - diazabicyclo[4.4.0]déc - 2 - ène - 4 - one, et ses isomères et racémates correspondants, composés selon la revendication 9.

15. La 2 - méthyl - 5 - (2 - chlorophényl) - 5 - [2 - [N,N - bis(1 - méthyl - éthyl)amino]éthyl] - 1,3 - diazabicyclo[4.4.0]décane - 4 - one, et ses isomères et racémates correspondants, composés selon la revendication 9.

16. Un composé selon la revendication 1, dans lequel $R_1$ est phényle.

17. La 2 - phényl - 5 - phényl - 5 - [2 - [N,N - bis(1 - méthyl - éthyl)amino]éthyl] - 1,3 - diazabicyclo[4.4.0]déc - 2 - ène - 4 - one, et ses isomères et racémates correspondants, composés selon la revendication 16.

18. La 2 - phényl - 5 - phényl - 5 - [2 - [N,N - bis(1 - méthyl - éthyl)amino]éthyl] - 1,3 - diazabicyclo[4.4.0]décane - 4 - one, et ses isomères et racémates correspondants, composés selon la revendication 16.

19. La 2 - phényl - 5(4 - phénylphényl) - 5 - [2 - [N,N - bis(1 - méthyl - éthyl)amino]éthyl] - 1,3 - diazabicyclo[4.4.0]déc - 2 - ène - 4 - one, et ses isomères et racémates correspondants, composés selon la revendication 16.

20. La 2 - phényl - 5 - (4 - phénylphényl) - 5 - [2 - [N,N - bis(1 - méthyl - éthyl)amino]éthyl] - 1,3 - diazabicyclo[4.4.0]décane - 4 - one, et ses isomères et racémates correspondants, composés selon la revendication 16.

21. La 2 - phényl - 5 - (2 - chlorophényl) - 5 - [2 - [N,N - bis(1 - méthyl - éthyl)amino]éthyl] - 1,3 - diazabicyclo[4.4.0]déc - 2 - ène - 4 - one, et ses isomères et racémates correspondants, composés selon la revendication 16.

22. La 2 - phényl - 5 - (2 - chlorophényl) - 5 - [2 - [N,N - bis(1 - méthyl - éthyl)amino]éthyl] - 1,3 - diazabicyclo[4.4.0]décane - 4 - one, et ses isomères et racémates correspondants, composés selon la revendication 16.